# EUROPEAN PATENT APPLICATION

(11) **EP 4 710 901 A1**
(43) Date of publication of application: **18.03.2026**
(21) Application number: 24200013.1
(22) Date of filing: 12.09.2024
(51) Int. Cl.: A61F 13/0206

(54) **A SUSTAINABLE MEDICAL DRESSING**

(71) Applicant: Mölnlycke Health Care AB, 431 21 Mölndal (SE)
(72) Inventor: FLACH, Jenny, 441 35 ALINGSÅS (SE); MADLUNG, Peter, 416 57 GÖTEBORG (SE)
(74) Representative: Kransell & Wennborg KB

(57) **Abstract**

The present disclosure generally relates to a sustainable medical dressing (1) comprising at least a backing layer (2) and an absorbent pad (3), wherein the backing layer (2) comprises a biodegradable nonwoven or biodegradable paper, and wherein the absorbent pad (3) comprises a superabsorbent material derived from a renewable source.

The present disclosure also relates to a medical dressing assembly (8) comprising the medical dressing (1).

## Description

### TECHNICAL FIELD

The present disclosure generally relates to a sustainable medical dressing comprising at least a backing layer and an absorbent pad. The medical dressing is environmentally friendly and is associated with a significantly reduced carbon footprint.

### BACKGROUND

Medical dressings are frequently used in wound care, both for the purpose of treating wounds and scars and for the purpose of preventing these from occurring in the first place. A medical dressing typically comprises an adhesive skin-contact layer arranged to contact the skin or wound of a patient, as well as a top layer, often referred to as a backing layer. In many cases, particularly when the dressing is to be applied to moderate or highly exuding wounds, the dressing further comprises a wound pad arranged between the backing layer and the adhesive skin-contact layer. To protect the dressing from contamination, a release liner may be arranged to cover the adhesive skin-contact layer.

Medical dressings are commonly composed of various synthetic materials, such as polyesters, polyurethanes, and synthetic superabsorbent polymers.

While these materials may provide strong fluid absorption, and form protective barriers against contamination, they typically fail to decompose naturally. Most of the synthetic materials used in conventional medical dressings are not biodegradable, and this may lead to significant waste accumulation that may remain in the environment for decades.

Furthermore, the production of synthetic materials often involves processes that are energy-intensive and rely on non-renewable resources, contributing to carbon emissions and depletion of finite resources. The disposal of non-biodegradable dressings typically requires incineration, which may trigger the release of toxic substances.

There is a growing consumer concern, as well as regulatory requirements towards reducing waste and enhancing sustainability in healthcare products. Environmentally conscious consumers and regulatory bodies generally demand "greener" alternatives in medical supplies.

There is consequently a need for a medical dressing which combines the advantages of traditional dressings, such as absorbency, breathability, contamination protection, comfort etc., with improved environmental friendliness. Such a dressing is preferably associated with a reduced carbon footprint.

### SUMMARY

In view of the above-mentioned problems, it is an object of the present disclosure to provide improvements in the field of medical dressings by reducing the environmental impact and the carbon footprint associated with traditional wound dressings.

According to a first aspect, there is provided a sustainable medical dressing comprising at least a backing layer and an absorbent pad, wherein the backing layer comprises a biodegradable nonwoven or biodegradable paper, and wherein the absorbent pad comprises a superabsorbent material derived from a renewable source.

The present disclosure presents a solution to the environmental challenges posed by traditional synthetic dressings. By replacing conventional synthetic materials with biodegradable and renewable materials, a significantly reduced environmental impact and carbon footprint is accomplished. The materials of the dressing may break down naturally in the environment after use, and thus avoid the problems of waste accumulation associated with non-biodegradable dressings.

Hence, a medical dressing of the present disclosure is configured to meet the stringent demands for sustainable medical products amongst conscious consumers and regulatory bodies.

The superabsorbent material is derived from a renewable source, such as cellulose, starch, chitin, etc. Such "natural" superabsorbents are capable of absorbing and retaining large amounts of wound exudate but represent sustainable and eco-friendly alternatives compared to their synthetic counterparts.

The backing layer comprises biodegradable paper or a biodegradable nonwoven.

Traditional backing layers are typically made from synthetic materials, such as polyesters and polyurethanes. However, these materials are not biodegradable. The use of a biodegradable backing layer minimizes the reliance on fossil fuels and reduces the emission of carbon dioxide.

Biodegradable nonwovens and papers can be engineered to offer comparable physical and mechanical properties to their synthetic counterparts, e.g. with respect to softness and flexibility.

In exemplary embodiments, the backing layer may comprise a biodegradable nonwoven comprising polylactic acid (PLA), polyhydroxyalkanoate (PHA), cellulose fibers, starch and/or polycaprolactone (PCL).

PLA and PHA are derived from renewable resources, such as corn starch and microbial fermentation, respectively. Both PLA and PHA provide good mechanical properties.

Cellulose fibers may be sourced from plants and provide good moisture management properties.

Starch is a low-cost biodegradable polymer that can be processed into nonwoven fabrics.

PCL is a biodegradable polyester with a slow degradation rate, which may suitably be used to structurally support the medical dressing.

In exemplary embodiments, the backing layer may comprise cellulose crepe paper.

Cellulose crepe paper is a variant of paper that has been crinkled to increase its surface area and elasticity. Cellulose crepe paper is flexible and stretchable and may conform well to the body during use. Compared to many synthetic materials, cellulose crepe paper is environmentally friendly and also cost-effective.

In exemplary embodiments, the superabsorbent material may be a cellulose-based superabsorbent material, preferably selected from carboxymethyl cellulose (CMC), carboxy ethyl cellulose, hydroxyethyl cellulose (HEC), bacterial cellulose, nanocellulose and/or crosslinked cellulose.

These cellulose-based superabsorbent materials may handle significant amounts of wound exudate, and maintain a balance between moisture absorption and retention, which is important for promoting wound healing. Furthermore, these materials are safe to use in contact with skin and wounds.

Carboxymethyl cellulose (CMC) is a water-soluble derivative of cellulose where hydrogen atoms on the hydroxyl groups of the glucose monomers are replaced with carboxymethyl groups. This modification enhances the cellulose's ability to absorb and retain water or exudate. Upon hydration, CMC forms a gel, and may provide a moist environment, which is beneficial for wound healing.

Carboxyethyl cellulose is similar to CMC, but with ethyl carboxyl groups attached.

Hydroxyethyl cellulose (HEC) is a non-ionic, water-soluble polymer derived by substituting hydroxyethyl groups into the cellulose backbone. It is generally thicker and more viscous compared to other cellulose ethers. HEC may stabilize the dressing structure and secure optimal moisture conditions.

Bacterial cellulose is produced by bacteria and is associated with a high absorbency and tensile strength.

Nanocellulose comprises nanosized cellulose fibrils with a high absorption capacity.

Crosslinked cellulose is cellulose that has undergone a chemical modification to create crosslinks between polymer chains to enhance its absorbency and stability. Crosslinked cellulose is beneficial in that it may maintain its structural integrity even when saturated with exudate.

In exemplary embodiments, the absorbent pad may comprise a mixture of cellulose fluff pulp and the superabsorbent material.

Cellulose fluff pulp is a highly absorbent, soft material derived from wood pulp. When combined with the superabsorbent materials described hereinbefore, the resulting composite material is suitable for managing moderate to highly exuding wounds. Fluff pulp absorbs liquid quickly, reducing the risk of leakage, while the superabsorbent material may retain the liquid, preventing re-wetting and keeping the wound dry.

An absorbent pad comprising cellulose fluff pulp and a superabsorbent material as described hereinbefore maintain an optimal moisture balance, and prevents the wound from drying out (which may delay the wound healing process). Wound exudate may be efficiently absorbed while simultaneously keeping the wound sufficiently moist. The risk of skin maceration around the wound is thereby significantly reduced. Furthermore, cellulose fluff pulp is soft and comfortable against the skin and remains flexible even when saturated.

Both cellulose fluff pulp and cellulose-based superabsorbent materials are derived from renewable resources and are biodegradable. In addition, cellulose fluff pulp is inexpensive, and the manufacturing cost of the medical dressing can thus be significantly reduced.

In exemplary embodiments, the absorbent pad may comprise at least a liquid acquisition layer and an absorbent layer; the absorbent layer comprising the superabsorbent material, wherein the liquid acquisition layer is void of superabsorbent material and is arranged below the absorbent layer.

The liquid acquisition layer is configured to quickly acquire and transfer wound exudate away from the wound and the skin towards the absorbent layer. The absorbent layer absorbs and retains the wound exudate and secures that the medical dressing can hold large volumes of exudate. This construction is beneficial to reduce the risk of maceration.

Furthermore, this construction keeps the superabsorbent material away from the wound site and prevents the superabsorbent material from direct contact with or entry into the wound, which could cause irritation of the wound. The layered pad construction prevents accumulation of exudate at the wound site and improves the overall liquid handling of the medical dressing.

In exemplary embodiments, the absorbent pad may comprise an airlaid cellulose-based nonwoven.

The "airlaid technology" involves laying down short cellulose fibers (and possibly other fibers or additives) in an air stream and then bonding them together, usually with a binder or by thermal means, to form a fabric-like structure without weaving or knitting. Airlaid cellulose-based nonwovens are highly absorbent, soft and flexible. Furthermore, airlaid nonwovens are breathable, and allow air to circulate through the dressing such that moisture may evaporate more efficiently from the backing layer.

It is also conceivable to include the superabsorbent material in the airlaid cellulose-based nonwoven.

In exemplary embodiments, the medical dressing may further comprise a barrier layer arranged between the backing layer and the absorbent pad, wherein the barrier layer is liquid impermeable and moisture permeable and comprises a biodegradable nonwoven.

The barrier layer may render the medical dressing more "leakage-proof'. The barrier layer may protect the medical dressing from penetration of external fluids (e.g. water from a patient taking a shower). However, the barrier layer still secures that moisture vapor from the wound exudate may be removed from the backing layer.

The barrier layer is preferably hydrophobic to prevent water and other liquids from passing therethrough.

In exemplary embodiments, the absorbent pad is defined by pad edges, and wherein the backing layer may be arranged to extend beyond the pad edges to form a border portion surrounding the absorbent pad, wherein the dressing further comprises an adhesive skin-contact layer arranged on at least the parts of the backing layer forming the border portion.

The backing layer has a bottom side facing the absorbent pad, and an opposing second side. The bottom side of the backing layer may comprise an adhesive skin-contact layer, e.g. an adhesive coating. The adhesive coating may anchor the absorbent pad to the bottom side of the backing layer. The adhesive areas of the backing layer surrounding the absorbent pad secure that the medical dressing may be anchored to the skin of a patient.

In alternative embodiments, the absorbent pad may be arranged between the backing layer and an adhesive skin-contact layer.

In other words, the adhesive skin-contact layer forms the bottom surface of the dressing.

The adhesive-skin contact layer may be an adhesive coating or it may be a layer or a film, preferably a biodegradable layer or film onto which an adhesive coating is provided.

Preferably the adhesive skin-contact layer comprises a water-based or biodegradable adhesive.

In a water-based adhesive, water is used as the primary solvent or dispersion medium for the adhesive components. A water-based adhesive is more environmentally friendly compared to solvent-based adhesives since they emit lower levels of volatile organic compounds (VOC).

A biodegradable adhesive may be formed from a renewable material, e.g. natural rubber latex, polylactic acid, starch, biobased polyurethane.

These adhesives are non-toxic, compatible with the human skin and are significantly more environmentally friendly than their synthetic counterparts.

In some embodiments, the medical dressing is non-adhesive.

Non-adhesive medical dressings may be used when frequent dressing changes are required. A non-adhesive medical dressing may be held in place with a secondary dressing or bandage. Alternatively, it may be attached to the skin by means of a separate, adhesive member, as will be described hereinbelow.

The medical dressing may comprise a skin-contact layer comprising cellulose fibers and/or alginate fibers.

Both cellulose and alginate are soft and comfortable and suitable for use in contact with fragile skin. Furthermore, bot cellulose and alginate are natural and biodegradable and associated with a reduced environmental impact.

Alginates are high molecular weight, hydrophilic polymers derived from seaweed. Alginate fibers are hydrophilic and form a gel in contact with aqueous fluids, e.g. wound exudate.

In exemplary embodiments, the skin-contact layer may comprise a mixture of cellulose fibers and alginate fibers.

A skin-contact layer comprising cellulose fibers and/or alginate fibers is suitably used when the medical dressing is non-adhesive.

According to another aspect, there is provided a medical dressing assembly comprising:
- a sustainable medical dressing as described hereinbefore; and
- a silicone-based adhesive member,
wherein the silicone-based adhesive member is configured to contact the skin or the wound of a wearer during use, and wherein the sustainable medical dressing is configured to be detachably attached to the silicone-based adhesive member.

A silicone-based adhesive is not biodegradable but is known to be skin-friendly and to have a very good adhesion to human skin. In the medical dressing assembly of the present disclosure, the silicone-based adhesive member is a separate component, which may remain on the skin while the medical dressing (arranged thereon) can be changed several times.

The medical dressing may be attached to the silicone-based member, and subsequently be detached and replaced with a new medical dressing. The silicone-adhesive member may thus be re-used several times with a plurality of different medical dressings. When the medical dressing has reached its full capacity, it may be removed and be discarded in an environmentally friendly manner (without any synthetic components present within the dressing).

In exemplary embodiments, the silicone-based adhesive member may be a skin-contact layer or a member having an annular shape.

The silicone-based adhesive member has a first, skin-facing side, and a second, opposing side. The second side may be provided with an adhesive to facilitate attachment of the medical dressing.

In embodiments where the silicone-based adhesive member has an annular shape, the adhesive member may be configured to encircle a wound; i.e. to form a closed circle around the wound site. At least the absorbent pad of the sustainable medical dressing may thus be arranged inside the annular silicone-based member.

The annular silicone-based adhesive member may be re-usable. Hence, after use, the adhesive member may be washed, sterilized and subsequently be re-applied onto the patient.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a sustainable medical dressing according to an exemplary embodiment of the present disclosure, as seen from the bottom side of the medical dressing.
Figure 2 schematically illustrates a partially split-view of a medical dressing according to another exemplary embodiment of the present disclosure.
Figure 3 schematically illustrates a split view of a medical dressing assembly comprising the medical dressing of figure 1 and an annular silicone-based adhesive member according to an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

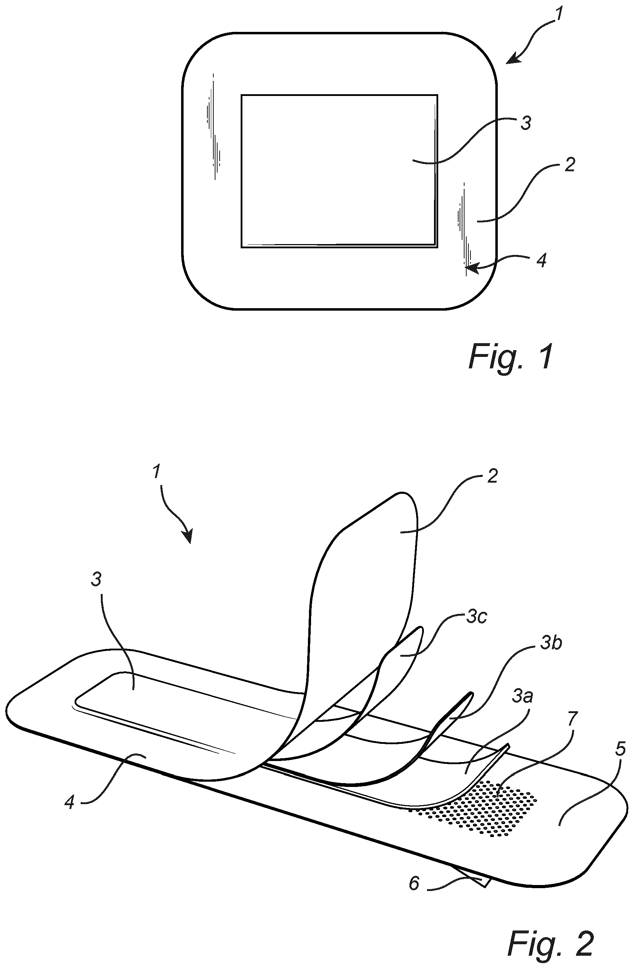

A medical dressing is schematically illustrated in figure 1. The sustainable medical dressing 1 comprises at least a backing layer 2 and an absorbent pad 3, wherein the backing layer 2 comprises a biodegradable nonwoven or biodegradable paper, and wherein the absorbent pad 3 comprises a superabsorbent material derived from a renewable source.

As used herein, the term "sustainable medical dressing" means a medical dressing in which the components (e.g. layers and materials) are derived from renewable and/or biodegradable materials. The sustainable medical dressing is designed to minimize the environmental impact through its lifecycle, i.e. from raw material through manufacturing, usage, and final disposal. The components of the medical dressing are made from materials capable of breaking down into natural elements (carbon dioxide, water, and biomass) without leaving any toxic residues.

In the context of the present disclosure, the "backing layer" is the top layer of the dressing, i.e. the outermost layer of the dressing. The backing layer is a continuous layer. In other words, the backing layer is void of perforations and incisions. The backing layer protects the layers of the dressing from potential contaminants. The backing layer is desirably a thin and pliable layer such that it can stretch and conform to the movement of a wearer. The backing layer comprises a biodegradable nonwoven or biodegradable paper.

As used herein, the term "biodegradable nonwoven" means a material made of fibers formed from renewable or natural polymers. The fibers may be bonded together by mechanical, thermal or chemical means. The biodegradable nonwoven is capable of decomposing naturally, without leaving any toxic residues.

For example, the biodegradable nonwoven may comprise polylactic acid (PLA), polyhydroxyalkanoate (PHA), cellulose fibers, starch and/or polycaprolactone (PCL).

Paper is typically biodegradable due to its natural fiber content, however its biodegradability can be compromised by additives, coatings or synthetic fiber mixtures. In the context of the present disclosure, the term "biodegradable paper" means a paper void of any synthetic fibers or non-biodegradable additives. In other words, the biodegradable paper does not contain any elements which may slow down or inhibit the decomposition of the paper. The biodegradable paper is composed of natural fibrous substrates derived from a cellulose-based source, e.g. wood.

The biodegradable paper may e.g. comprise cellulose crepe paper. Cellulose crepe paper is advantageous in that it is flexible and stretchable and may conform well to the body during use.

The absorbent pad 3 comprises a superabsorbent material derived from a renewable source.

As used herein, the term "superabsorbent material" means a material capable of absorbing at least 20 times its own weight in aqueous fluids. The superabsorbent material may retain the aqueous fluid under moderate pressure.

That the superabsorbent material is "derived from a renewable source" means that the superabsorbent is formed from renewable resources including plant-based materials, animal products or microbial products.

The superabsorbent material may comprise starch-graft polymers, polylactic acid (PLA), polyhydroxylalkanoate (PHA), and/or polyvinyl alcohol (PVOH).

Typically, the superabsorbent material is a cellulose-based superabsorbent material. The cellulose-based superabsorbent material may be selected from carboxymethyl cellulose (CMC), carboxy ethyl cellulose, hydroxyethyl cellulose (HEC), bacterial cellulose, nanocellulose or crosslinked cellulose.

The absorbent pad 3 may comprise a mixture of cellulose fluff pulp and the superabsorbent material.

The absorbent pad is thus highly absorbent, yet soft and conformable. Furthermore, such an absorbent pad is generally inexpensive to manufacture.

As best illustrated in figure 2, the absorbent pad may comprise more than one layer. A layered pad construction may be preferred to optimize the liquid handling properties of the medical dressing.

For example, the absorbent pad 3 may comprise at least a liquid acquisition layer 3a and an absorbent layer 3b; the absorbent layer 3b comprising the superabsorbent material, wherein the liquid acquisition layer 3a is void of superabsorbent material and is arranged below the absorbent layer.

As illustrated in figure 2, the liquid acquisition layer 3a forms the lowermost layer of the absorbent pad 3. This arrangement is beneficial to prevent leakage of superabsorbent towards the wound site. The liquid acquisition layer may e.g. comprise a biodegradable nonwoven, e.g. a cellulose-based nonwoven.

In figure 2, the medical dressing further comprises a third layer 3c. The third layer 3c may be a liquid spreading layer or an additional absorbent layer.

For example, the third layer may be a biodegradable nonwoven or a layer comprising the superabsorbent material and/or cellulose fluff pulp.

The absorbent pad may comprise an airlaid cellulose-based nonwoven.

For example, the liquid acquisition layer 3a may comprise an airlaid cellulose-based nonwoven.

In some embodiments, the medical dressing may further comprise a barrier layer (not shown) arranged between the backing layer 2 and the absorbent pad 3, wherein the barrier layer is liquid impermeable and moisture permeable and comprises a biodegradable nonwoven.

The purpose of the barrier layer is to enhance the leakage-barrier properties of the medical dressing, e.g. to prevent water from entering the medical dressing.

The barrier layer is preferably hydrophobic. The barrier layer may e.g. comprise a hydrophobic coating. For example, the barrier layer may comprise polylactic acid (PLA).

As illustrated in figure 1 (and in figure 2), the absorbent pad 3 is defined by pad edges, and wherein the backing layer 2 is arranged to extend beyond the pad edges to form a border portion 4 surrounding the absorbent pad 3, wherein the dressing further comprises an adhesive skin-contact layer 5 arranged on at least the parts of the backing layer 2 forming the border portion 4.

The backing layer 2 has a top side and a bottom side, wherein the bottom side is arranged to face the skin during use.

In figure 1, the dressing 1 is seen from the bottom side. The adhesive skin-contact layer may be an adhesive coating arranged on the bottom side of the backing layer 2. The adhesive coating is used to anchor the absorbent pad to the backing layer. The adhesive areas of the backing layer surrounding the absorbent pad may be used to anchor the medical dressing onto the skin.

As best illustrated in figure 2, the absorbent pad 3 may be arranged between the backing layer 2 and an adhesive skin-contact layer 5.

Accordingly, the adhesive skin-contact layer 5 forms the bottom surface; i.e. skin-contact surface of the medical dressing. In the dressing illustrated in figure 2, the adhesive skin-contact layer 5 and the backing layer 2 form the border portion 4 of the medical dressing.

The adhesive skin-contact layer 5 may comprise a plurality of perforations 7 to secure efficient liquid acquisition and liquid handling. As illustrated in figure 2, the adhesive skin-contact layer 5 comprises a plurality of perforations 7 in the area underlying the absorbent pad 3, but is void of perforations in the area forming the border portion 4.

The lack of perforations in the border portion 4 may enhance the adherence of the dressing to the skin such that the dressing may stay on the skin for prolonged periods.

Preferably, the adhesive skin-contact layer comprises water-based adhesive or a biodegradable adhesive.

The water-based adhesive may comprise at least 50%, such as at least 70% by weight of water.

Examples of water-based adhesives include polyvinyl acetate (PVA) adhesives, acrylic emulsion adhesives, dextrin-based adhesives, latex adhesives, ethylene vinyl acetate (EVA) emulsions.

A biodegradable adhesive is configured to break down into natural, non-toxic components, such as water, carbon dioxide, and biomass. Examples of biodegradable adhesives include starch- and dextrin-based adhesives, cellulose-based adhesives (e.g. derived from carboxymethyl cellulose or hydroxyethyl cellulose), natural waxes, biodegradable polyvinyl acetates and biodegradable polyester-polyurethane blends (wherein the polyester component is designed to be eco-friendly and the polyurethane part is aliphatic).

In some embodiments, the medical dressing may be non-adhesive.

In such embodiments, the surface area of the absorbent pad generally corresponds to the surface area of the backing layer. In other words, the backing layer and the absorbent pad may be coextensive in length and width. The non-adhesive medical dressing may be combined with a secondary dressing or a separate adhesive component that may anchor the medical dressing to the skin.

The medical dressing may further comprise a skin-contact layer comprising cellulose fibers and/or alginate fibers.

Such a skin-contact layer is typically used in embodiments where the medical dressing is non-adhesive or has the construction illustrated in figure 1. In figure 1, the medical dressing is adhesive in the border portion, but the absorbent pad is arranged to contact the skin. A skin-contact layer comprising cellulose-fibers and/or alginate fibers may form the lowermost layer of the absorbent pad and be configured to be arranged in skin-contact.

The medical dressing typically further comprises a release liner detachably attached to the adhesive skin-contact layer. The release liner may comprise one or a plurality of release liner portion and is configured to cover the entire surface of the adhesive skin-contact layer 5. In figure 2, a portion of the release liner is denoted 6.

The release liner is typically a paper comprising a biodegradable coating. For example, the coating may comprise a wax, e.g. beeswax.

The release liner is typically adhesively attached to a skin-contact surface of the medical dressing. The skin-contact surface may be formed by the adhesive skin-contact layer and/or the absorbent pad.

The medical dressing may further comprise an antimicrobial or skin/wound beneficial agent selected from honey, aloe vera, chitosan, and/or tea tree oil.

These agents may promote wound healing and are also considered environmentally friendly due to their natural origin and biodegradability.

Honey is a natural antimicrobial having anti-inflammatory and wound healing promoting properties. Aloe vera is moisturizing and may reduce pain and inflammation, as well as stimulate skin repair. Chitosan has hemostatic properties and is antimicrobial and may thus be beneficial to promote wound healing. Tea tree oil has an antimicrobial effect and is suitable for preventing infection.

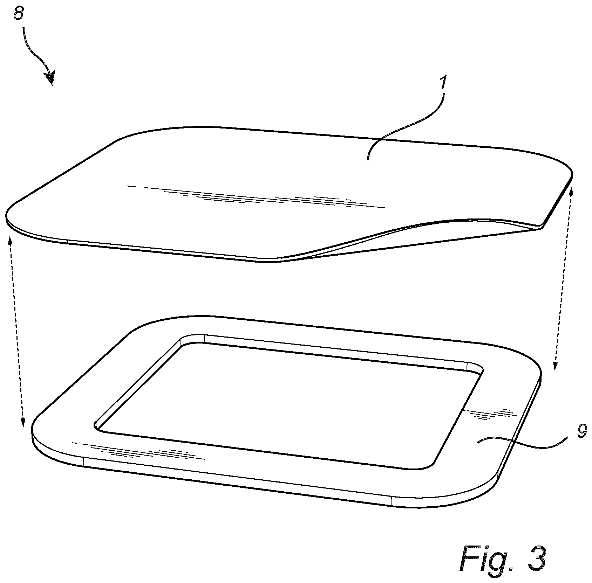

Figure 3 schematically illustrates a medical dressing assembly 8 comprising:
- a sustainable medical dressing 1 as described hereinbefore; and
- a silicone-based adhesive member 9,
wherein the silicone-based adhesive member 9 is configured to contact the skin or the wound of a wearer during use, and wherein the sustainable medical dressing 1 is configured to be detachably attached to the silicone-based adhesive member 9.

In figure 3, the silicone-based adhesive member 9 has an annular shape. However, it is equally conceivable that the silicone-based adhesive member 9 is a separate adhesive skin-contact layer.

The silicone-based adhesive member 9 may remain on the skin whilst the medical dressing 1 is changed and replaced several times. In this regard, the medical dressing may be discarded separately in an environmentally friendly manner.

Compared to traditional medical dressings, where a synthetic adhesive (e.g. silicone) forms an integrated layer in the medical dressing, significantly less of such synthetic components are required in the medical dressing assembly of the present disclosure.

As illustrated in figure 3, the adhesive member may be configured to encircle a wound; i.e. to form a closed circle around the wound site. At least a portion of the sustainable medical dressing 1 may thus be arranged inside the annular silicone-based member.

The medical dressing 1 may be a border dressing, i.e. a dressing comprising a wound pad and a border portion surrounding the wound pad. The shape of the wound pad (not shown) of the medical dressing may thus match the shape of the interior space formed by the annular silicone-based adhesive member 9. The border portion of the medical dressing may be adhesively attached to the annular silicone-based adhesive member 9.

The silicone-based adhesive member 9 has a first, skin-facing side, and a second, opposing side. The second side may be provided with an adhesive to facilitate attachment of the medical dressing 1.

The medical dressing may have a maximum lateral (x) extension, i.e. width, corresponding to the maximum lateral (x) extension of the annular silicone-based member 9, and a maximum longitudinal (y) extension, i.e. length corresponding to the maximum longitudinal (y) extension of the annular silicone-based member 9.

Alternatively, the maximum lateral (x) extension and/or maximum longitudinal (y) extension of the medical dressing is larger than the maximum lateral (x) extension and/or the maximum longitudinal (y) extension of the annular silicone-based member 9.

The annular silicone-based adhesive member 9 may be re-usable. Hence, after use, the adhesive member may be washed, sterilized and subsequently be re-applied onto the patient.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A sustainable medical dressing (1) comprising at least a backing layer (2) and an absorbent pad (3), wherein said backing layer (2) comprises a biodegradable nonwoven or biodegradable paper, and wherein said absorbent pad (3) comprises a superabsorbent material derived from a renewable source.

2. The sustainable medical dressing (1) according to claim 1, wherein said backing layer (2) comprises a biodegradable nonwoven comprising polylactic acid (PLA), polyhydroxyalkanoate (PHA), cellulose fibers, starch and/or polycaprolactone (PCL).

3. The sustainable medical dressing (1) according to claim 1, wherein said backing layer (2) comprises cellulose crepe paper.

4. The sustainable medical dressing (1) according to any one of the preceding claims, wherein said superabsorbent material is a cellulose-based superabsorbent material, preferably selected from carboxymethyl cellulose (CMC), carboxy ethyl cellulose, hydroxyethyl cellulose (HEC), bacterial cellulose, nanocellulose or crosslinked cellulose.

5. The sustainable medical dressing (1) according to any one of the preceding claims, wherein said absorbent pad (3) comprises a mixture of cellulose fluff pulp and said superabsorbent material.

6. The sustainable medical dressing (1) according to any one of the preceding claims, wherein said absorbent pad (3) comprises at least a liquid acquisition layer (3a) and an absorbent layer (3b); said absorbent layer (3b) comprising said superabsorbent material, wherein said liquid acquisition layer (3a) is void of superabsorbent material and is arranged below said absorbent layer.

7. The sustainable medical dressing (1) according to any one of the preceding claims, wherein said absorbent pad (3) comprises an airlaid cellulose-based nonwoven.

8. The sustainable medical dressing (1) according to any one of the preceding claims, wherein said medical dressing further comprises a barrier layer arranged between said backing layer (2) and said absorbent pad (3), wherein said barrier layer is liquid impermeable and moisture permeable and comprises a biodegradable nonwoven.

9. The sustainable medical dressing (1) according to any one of the preceding claims, wherein said absorbent pad (3) is defined by pad edges, and wherein said backing layer (2) is arranged to extend beyond said pad edges to form a border portion (4) surrounding said absorbent pad (3), wherein said dressing further comprises an adhesive skin-contact layer (5) arranged on at least the parts of said backing layer (2) forming said border portion (4).

10. The sustainable medical dressing (1) according to any one of the preceding claims, wherein said absorbent pad (3) is arranged between said backing layer (2) and an adhesive skin-contact layer (5).

11. The sustainable medical dressing (1) according to claim 9 or claim 10, wherein said adhesive skin-contact layer (5) comprises a water-based adhesive or a biodegradable adhesive.

12. The sustainable medical dressing (1) according to any one of claims 1-8, wherein said medical dressing is non-adhesive.

13. The sustainable medical dressing (1) according to claim 12, further comprising a skin-contact layer comprising cellulose fibers and/or alginate fibers.

14. A medical dressing assembly (8) comprising:
- a sustainable medical dressing (1) according to any one of claims 1-13; and
- a silicone-based adhesive member (9),
wherein said silicone-based adhesive member (9) is configured to contact the skin or the wound of a wearer during use, and wherein said sustainable medical dressing (1) is configured to be detachably attached to said silicone-based adhesive member (9).

15. The medical dressing assembly (8) according to claim 14, wherein said silicone-based adhesive member (9) is a skin-contact layer or a member having an annular shape.
